# EUROPEAN PATENT SPECIFICATION

(11) **EP 2 247 301 B1**
(45) Date of publication and mention of the grant of the patent: **11.06.2014**
(21) Application number: 09712807.8
(22) Date of filing: 19.02.2009
(51) Int. Cl.: A61P 35/00

(54) **USE OF THERAPEUTIC FUSION PEPTIDES FOR THE TREATMENT AND PREVENTION OF CANCER**
VERWENDUNG VON THERAPEUTISCHEN FUSIONSPEPTIDEN ZUR BEHANDLUNG UND VORBEUGUNG VON KREBS
UTILISATION DE PEPTIDES DE FUSION THÉRAPEUTIQUES POUR LE TRAITEMENT ET LA PRÉVENTION D'UN CANCER

(30) Priority: 20.02.2008 US 30021
(43) Date of publication of application: 10.11.2010
(73) Proprietor: Arizona Biomedical Research Commission, Phoenix, AZ 85007 (US)
(72) Inventor: SCHROEDER, Joyce A., Tucson Arizona 85750 (US)
(74) Representative: Tombling, Adrian George
(86) International application number: PCT/US2009/034541
(87) International publication number: WO 2009/105557

(56) References cited:
- WO-A-02/058450
- WO-A-2004/092339
- WO-A-2005/042573
- WO-A-2006/113667
- SCHROEDER J A ET AL: "MUC1 alters [beta]-catenin-dependent tumor formation and promotes cellular invasion" ONCOGENE 20030306 GB, vol. 22, no. 9, 6 March 2003 (2003-03-06), pages 1324-1332, XP002537486 ISSN: 0950-9232 cited in the application
- TETSU O ET AL: "[beta]-catenin regulates expression of cyclin D1 in colon carcinoma cells" NATURE 19990401 GB, vol. 398, no. 6726, 1 April 1999 (1999-04-01), pages 422-426, XP002537483 ISSN: 0028-0836 cited in the application
- CHOI YOUNG-JIN ET AL: "Erlotinib prevents pulmonary metastasis in curatively resected breast carcinoma using a mouse model" ONCOLOGY REPORTS, vol. 16, no. 1, July 2006 (2006-07), pages 119-122, XP002537484 ISSN: 1021-335X
- FRIESS T ET AL: "Combination treatment with erlotinib and pertuzumab against human tumor xenografts is superior to monotherapy" CLINICAL CANCER RESEARCH 20050715 US, vol. 11, no. 14, 15 July 2005 (2005-07-15), pages 5300-5309, XP002537485 ISSN: 1078-0432
- BITTER B G ET AL: "Intracellular MUC1 peptides inhibit cancer progression" CLINICAL CANCER RESEARCH 20090101 US, vol. 15, no. 1, 1 January 2009 (2009-01-01), pages 100-109, XP002538349 ISSN: 1078-0432

## Description

### TECHNICAL FIELD OF THE INVENTION

This invention is related to the area of cancer therapeutics and prophylactics. In particular, it relates to methods of inhibiting, retarding, and reducing risk of cancer initiation, growth, and invasion.

### BACKGROUND OF THE INVENTION

MUC1 (DF3, CD227, episialin, POEM) is a heavily O-glycosylated heterodimeric protein of >300 kDa, normally expressed abundantly on the apical surface of glandular epithelia. In greater than 90% of human breast carcinomas and metastases, apical localization is lost and MUC1 is overexpressed (by greater than 10 fold) and underglycosylated (1, 2). Deregulated expression of MUC1 is found in many other types of adenocarcinomas as well, including cancers of the lung, pancreas, ovary and prostate, in addition to being highly expressed in leukemias, myelomas and lymphomas (3-5). Studies in both genetic mouse models and cell line models have demonstrated that MUC1 is an oncogene. A transgenic mouse model driving MUC1 (human) overexpression to the mouse mammary gland (MMTV-MUC1) results in the development of breast cancer and is accompanied by a failure of the mammary gland to undergo complete postlactational regression via apoptosis (6). Transfection of MUC1 into 3Y1 fibroblasts induces their transformation, and transfection of MUC1 into colon cancer cells demonstrates that MUC1 overexpression inhibits drug-induced apoptosis (7).

The cytoplasmic domain of MUC1 contains sites for multiple protein interactions, although these interactions go largely unformed in the polarized epithelium of the normal breast, as the binding partners of MUC1 are typically found on the basolateral membrane (Reviewed in (8) and (9, 10)). During cancer progression, when there is a loss of cellular polarization, MUC1 is overexpressed and functionally interacts with src, GSK3β, epidermal growth factor receptor (EGFR) and β-catenin, among others (9, 11, 12). The sites for interaction between MUC1 and these proteins have been mapped to distinct domains within the 72-amino acid cytoplasmic tail of MUC1 (11, 13-15). Both EGFR and src can phosphorylate MUC1 on a YEKV motif, and this phosphorylation results in increased binding of MUC1 to β-catenin through an SAGNGGSSLS domain (11). Recent evidence demonstrates that the interaction between MUC1 and EGFR can significantly modulate EGFR biology and effect EGFR-dependent transformation {Pochampalli, 2007 #537; Pochampalli, 2007 #527}.

The Epidermal Growth Factor Receptor (EGFR) family of tyrosine kinases are frequently deregulated in cancer, and commonly amplified and/or overexpressed in invasive carcinoma [reviewed in (16)]. The family is comprised of four homologous type 1 tyrosine kinase receptors (including EGFR, her2/neu/erbB2, erbB3 and erbB4) and multiple related ligands [including epidermal growth factor (EGF) and transforming growth factor alpha (TGFα), among others]. Ligand-induced receptor homo- or hetero-dimerization results in tyrosine kinase activation and transphosphorylation of tyrosine residues in the cytoplasmic domain. This leads to the recruitment of a variety of effector proteins including Src, PI 3-kinase, Shc, PLCγ, STATs, Grb2, and cbl, resulting in proliferation, migration, inhibition of apoptosis, differentiation, or degradation of endocytosed receptors (17-20).

It has been established in both human breast cancer cell lines and transgenic mice overexpressing MUC1 (MMTV-MUC1) that MUC1 and EGFR biochemically interact, resulting in the potentiation of EGF-dependent p42/44 ERK activation during lactation (11, 21). Recently, our laboratory has demonstrated that MUC1 expression inhibits the ligand-mediated ubiquitination and degradation of EGFR while enhancing its internalization and recycling (Pochampalli et al 2007). To evaluate the role of Muc1 in transformation, we further generated WAP-TGFα mice on a Muc1 null background which revealed that Muc1 expression has a dominant effect on TGFα-dependent transformation of the breast, promoting both onset and progression (Pochampalli et al 2007b).

While it is now established that EGFR is potently regulated by MUC1 expression, transgenic mouse models have also implicated the cell adhesion protein, β-catenin, in EGFR and MUC1 signaling. In a study of the WAP-TGFα transgenic model, Wnt1 and Wnt3 were found to be selectively activated in the most aggressive breast tumors (22). The Wnts are secreted glycoproteins that bind the transmembrane frizzled receptor, resulting in a signaling cascade that inactivates the mechanism for β-catenin degradation and results in transformation (23-28). Additionally, in MMTV-Wnt1 transgenic mice, EGFR was found to interact with and phosphorylate β-catenin in a tumor-specific manner (Schroeder et al 2002). These studies demonstrate that β-catenin and EGFR can affect their respective pathways to promote transformation. Finally, MUC1 is also implicated in β-catenin dependent transformation, indicating that these three proteins have the ability to cooperatively promote cancer progression. In MMTV-Wnt-1 transgenic mouse models crossed onto a Muc1-null background, loss of Muc1 corresponds to a significant reduction in tumor progression (Schroeder et al, 2003). Additionally, interactions between MUC1 and β-catenin were found to be highly increased in samples from human metastatic breast tumors, indicating that these interactions are clinically relevant (Schroeder et al 2003).

Together, these studies demonstrate the strong potential for MUC1, EGFR and β-catenin to affect each other during transformation, including their striking co-upregulation during transformation and metastasis. MUC1 can inhibit the downregulation of EGFR and promote the transforming ability of both EGFR and β-catenin, and genetically derived mouse models implicate MUC1 in both EGFR- and β-catenin-dependent transformation and metastasis. Interestingly, the interaction sites on MUC1 for both EGFR and β-catenin lie in tandem on the MUC1 cytoplasmic domain.

There is a continuing need in the art to develop treatments that are effective in preventing and treating cancers. WO2006/113667 teaches the use of a fusion peptide which interrupts the interaction between MUC1 and β-catenin and can be used to treat an established cancer. WO2004/092339 teaches compositions and methods for inhibiting the binding of the carboxy-terminus of MUC1 to PD2 domains and to enhance the sensitivity of MUC1 expressing cancer cells to chemotherapeutic agents.

### SUMMARY OF THE INVENTION

According to one aspect of the invention there is provided a fusion peptide according to claim 1 for use in treating a human who has an identified elevated risk of cancer. The probability of initiation of the cancer is thereby reduced.

According to another aspect of the invention there is provided a fusion peptide according to claim 6 and an EGFR inhibitor for use in treating a human who has had a tumor resected. The probability of recurrence or metastasis of the tumor is thereby reduced.

According to yet another aspect of the invention there is provided a fusion peptide according to claim 10 for use in treating a patient with skin cancer. The invasiveness or growth of the cancer is thereby reduced or retarded.

These and other aspects which will be apparent to those of skill in the art upon reading the specification provide the art with new methods for treating or preventing cancers.

### BRIEF DESCRIPTION OF THE DRAWINGS

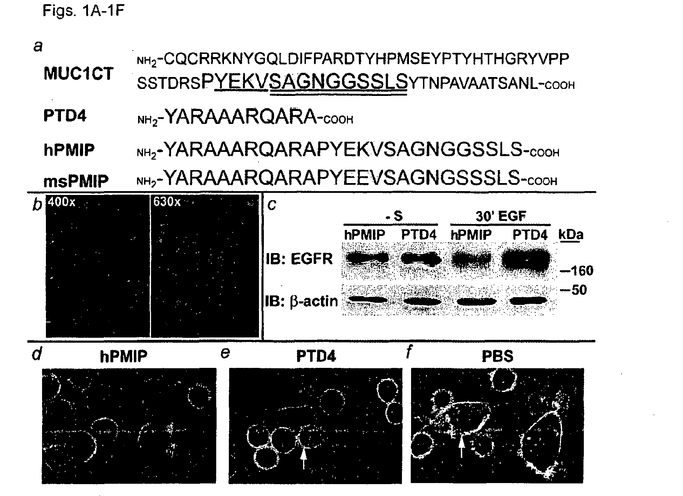

Figs. 1A-1F. A MUC1 mimetic peptide (PMIP) can efficiently enter cells, promote EGFR degradation and inhibit the interaction of MUC1 and β-catenin. Fig. 1A) Human MUC1's cytoplasmic amino acid sequence (MUC1CT; SEQ ID NO: 15), which includes an EGFR/src phosphorylation site (underline; SEQ ID NO: 15, residues 46-49) and β-catenin binding site (double underline; SEQ ID NO: 15, residues 50-59). The PTD4 10ng/ml) or without (-S, no serum) and protein lysates were generated and separated on an SDS-PAGE. The lysates were immunoblotted (IB) for EGFR (1005) and β-actin (AC-15) Fig. 1*D-F* MDA-MB-468 cells were treated with EGF (10ng/ml) and either (Fig. 1*D*) hPMIP (10µM), (Fig. 1*E*) PTD4 (10µM) or (Fig. 1*F*) PBS overnight. The cells were fixed, probed for MUC1 (Texas Red) and β-catenin (FITC) and examined on a confocal microscope (400x). Co-localization (arrows) analysis was performed using Image Pro Plus and is designated with white pixels.

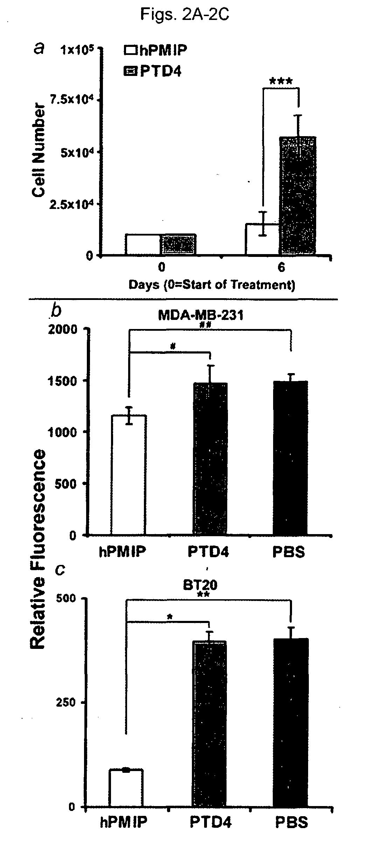

Figs. 2A-2C. PMIP inhibits cell proliferation and invasion of breast cancer cells *in vitro.* Fig. 2*A*) BT20 cells were cultured in a 96-well dish (10⁴ cells/well) and treated daily for 6 days with either hPMIP (10µM) or PTD4 (10µM) in RPMI 1640/1% FBS. An MTT assay was performed to quantify cell number after treatment was complete (***, p<0.0001). Error bars represent standard error. Fig. 2*B*) MDA-MB-231 and Fig. 2*C*) BT20cell lines were treated with either hPMIP (50µM), PTD4 (50µM), or PBS overnight, labeled with Calcein AM, allowed to invade through a Transwell (8.0µM) insert into a Type I collagen gel, and the invaded cells were fluorescently measured. (ANOVA, *p<0.0001,** p<0.0001 , #p= 0.007, ##p= 0.007). Error bars represent standard deviation.

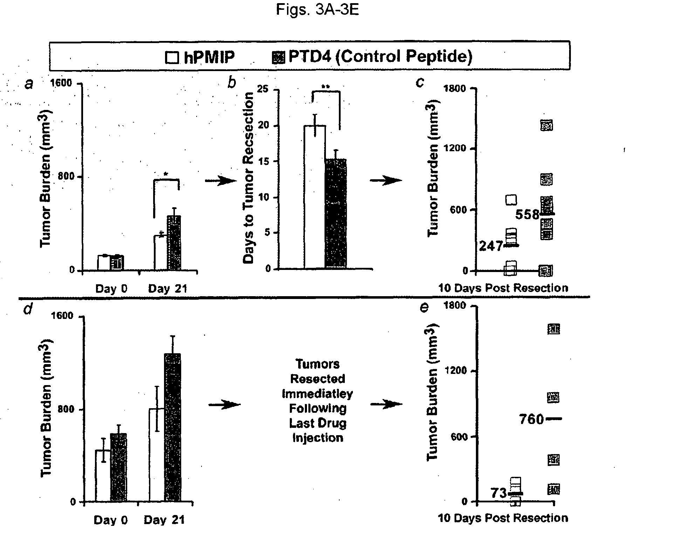

Figs. 3A-3E. PMIP significantly inhibits tumor growth and recurrence *in vivo.* Fig. 3*A*) MDA-MB-231 cells in Matrigel were injected into the mammary fat pad of *scid* mice, and daily peptide treatment (50µg/g body weight of hPMIP or PTD4) began when tumors reached 100mm³ (PTD4 and hPMIP n=8) and primary tumor growth was assessed (*, p=0.028). Fig. 3*B*) After the end of treatment, the amount of time the tumors took to progress to 1000mm³ was measured (ANOVA, **, p=0.03). Fig. 3*C* After resection, mice were observed for tumor regrowth at the primary site or secondary mammary glands (PTD4 n=8 and hPMIP n=7). Fig. 3*D*) MDA-MB-231 cells in Matrigel were injected into the mammary fat pad of *scid* mice, and daily peptide treatment (50µg/g of hPMIP or PTD4) began when tumors reached 500mm³ (PTD4 n=4 and hPMIP n=6) and primary tumor growth was assessed Fig. 3*E*) Following 21 days of treatment the tumors were resected immediately and tumor regrowth at the primary site and spread to secondary mammary glands was monitored (PTD4 n=4 and hPMIP n=4). Error bars represent standard error.

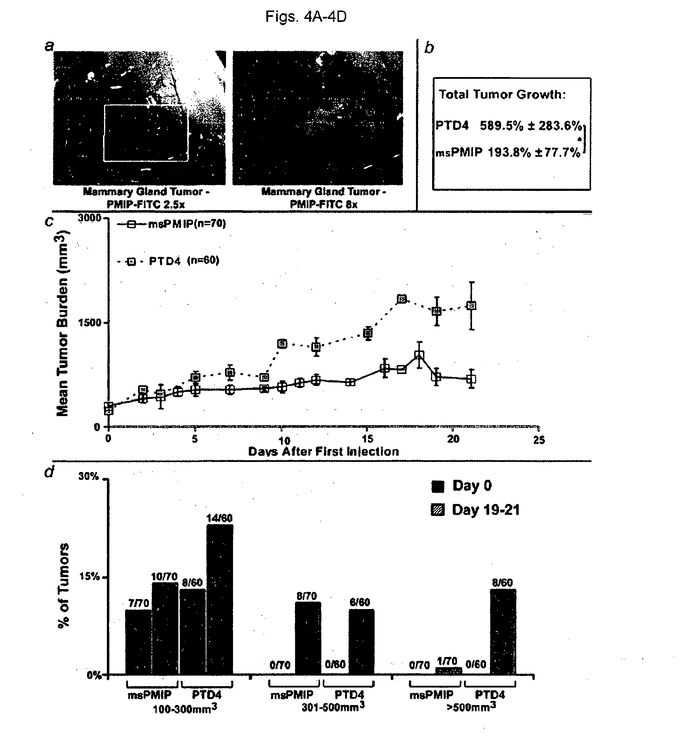

Figs. 4A-4D. PMIP significantly slows progression of MMTV-pyV mT induced mammary gland tumors. Fig. 4*A*) MMTV-pyV mT transgenic mice were injected with FITC-msPMIP (50µg/g body weight), sacrificed four hours later and various tissues visualized using fluorescence microscopy. FITC-msPMIP localization in the mouse mammary gland tumors is shown (2.5x and 8x). Fig. 4*B*) Mammary gland tumors (>0.5 cm in diameter) were allowed to develop and mice were injected daily (50µg/g body weight, 21 day treatment, i.p. injection, 1X per day) with either msPMIP (7 mice) or PTD4 (6 mice). At the end of treatment, animals were sacrificed and proteins lysates made of the tumors for later analysis. In the course of treatment, total tumor growth for all tumor sites (msPMIP n=70, PTD4 n=60) was significantly lower in the msPMIP treated mice than in the PTD4 mice (193.8% ± 77.7% vs. 589.5% ± 283.6%, *ANOVA p=0.039). Fig. 4*C*) Mammary gland tumors of msPMIP treated mice grew at a significantly slower rate than PTD4 treated tumors (ANOVA p=0.0076). Fig. 4*D*) Tumor size distribution for the msPMIP or PTD4 treated transgenic mice revealed that 47% (28 out of 60 possible tumor sites) of the PTD4 treated tumors were larger than 100mm³ compared to 27% (19 out of 70 possible tumor sites) of the msPMIP treated tumors. Numbers above data are number of tumors that meet the size criteria over the total potential tumor sites.

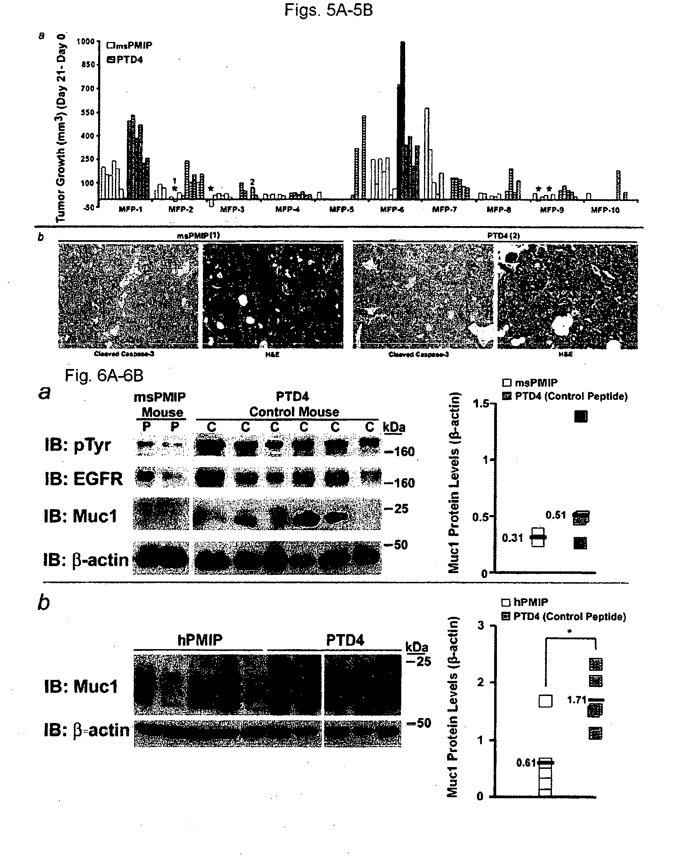

Figs. 5A-5B. MMTV-pyV mT tumors have differential response to msPMIP. Fig. 5*A*) Individual growth of each tumor site (each bar is a mammary fat pad/tumor site from one mouse) from animals described in Fig. 3 were treated daily with msPMIP (Dark grey) or PTD4 (Light grey) at 50µg/g body weight for 21 days. Tumor progression was observed every three days during the 21 day treatment (PTD4, n=60 and PMIP, n=70). In four instances (*), msPMIP treated tumors completely regressed, however none of the control (PTD4) treated tumors regressed. MFP=mammary fat pad Fig. 5*B*) Tumors from msPMIP (1, left 2 panels) and PTD4 (2, right 2 panels) treated mice were sectioned (3µm) and subsequently used for hemotoxylin-eosin staining and cleaved caspase-3 immunohistochemistry (200x).

Figs. 6A-6B. PMIP is associated with reduced Muc1 expression. Fig. 6*A*) A representative MMTV- pyV mT msPMIP (P) treated mouse and a PTD4 mouse (C) were each injected 30 minutes prior to sacrifice with epidermal growth factor (1µg/g body weight) and with peptide. Following sacrifice the tumors were collected and protein lysates were generated. Protein (50µg) was separated by SDS-PAGE, transferred and immunoblotted for expression of phosphotyrosine (PY99), EGFR (1005), Muc1 (CT2), and β-actin (AC-15). Fig. 6*B*) Lysates from MDA-MD-231 xenograft tumors (not treated with EGF; described in Fig. 2) were similarly analyzed to determine levels of EGFR and MUC1 protein expression. Relative protein levels of Muc1 were measured by densitometry and graphed (Muc1/β-actin, ANOVA, *p=0.014). Molecular weights are shown on the right. IB = immunoblot. White lines through blots indicate same gel and exposure but were non- contiguous.

### DETAILED DESCRIPTION OF THE INVENTION

It is a discovery underlying the present invention that MUC1 mimetic peptides are selectively retained in mammary gland tumors, colon, and skin after systemic administration. Moreover, MUC1 mimetic peptides reduce tumor initiation. In addition, MUC1 mimetic peptides can be used in conjunction with other anti-EGFR treatments in the adjuvant context, i.e., after surgery.

The fusion peptide for use according to the invention has the structure A-B-C or C-B-A, wherein A is a protein transduction domain which enhances translocation of attached macromolecules across cellular membranes selected form the group consisting of SEQ.ID.N. 3-6. B is a spacer of 0-5 amino acid residues. C is a polypeptide of 6-15 amino acid residues. C comprises all or a portion of PYEKVSAGNGGSSLS (SEQ ID NO: 1). The portion of C comprises GGSSLS (SEQ ID NO: 2). Alternatively, at least one of said 6-15 amino acid residues is conservatively substituted such that an uncharged polar amino acid replaces an uncharged polar amino acid, or a non-polar amino acid replaces a non-polar amino acid residue, or an acidic amino acid replaces an acidic amino acid, or wherein one of said 6- 15 amino acid residues is substituted with an A residue.

Any protein transduction domain (PTD) can be used in fusion proteins. These include any of the domains which have been previously identified and used for protein transduction. See for example the extensive Table 1 of Dietz and Bahr, Molecular and Cellular Neuroscience, 27 (2004) 85-131. Certain of such domains are shown in SEQ ID NO: 3, 4, 5, and 6. Other PTDs including synthetic ones can be used. These domains facilitate the uptake by the cells of the attached peptides.

Spacers for use in fusion protein are additional amino acid residues that are used in fusion proteins typically to facilitate manufacture or synthesis. These can be fairly innocuous and are of a length of from 0 to 5 residues. The linkers can be monotonous or mixed residue. The residues can be random or sequences obtained from other proteins or designed for a particular property, *e.g*., physical, chemical or biological.

MUC1 cytoplasmic domain peptides such as SEQ ID NO: 14 increase invasion of breast cancer cells. Schroeder, 2003. Surprisingly, shorter portions of such peptides actually have the opposite effect. These peptides comprises from 6 to 15 contiguous amino acid residues selected from SEQ ID NO: 1 and include the amino acid sequence shown in SEQ ID NO: 2. Slight deviations from the precise sequence may be used to optimize activity, such as by substitution of one, two or three residues to make conservative changes or by substitution with alanine. Conservative changes substitute similar residues for each other, such as an uncharged polar for an uncharged polar, or a non-polar for a non-polar, or an acidic for an acidic residue. Thus G or S residues can be substituted with G, S, T, C, Y, N, and Q. L residues can be substituted with A, V, I, P, F, W, and M. A, V, and P residues can be substituted with A, V, L, I, P, F, W, and M residues. Y or N residues can be substituted with G, S, T, C, Y, N, and Q residues. E residues can be substituted with a D residue. K residues can be substituted with an R or H residue. Any residue can be substituted with an alanine residue unless such substitution is found to destroy the invasion and metastasis inhibiting activity. Such substituted peptides can be readily tested, e.g., using invasion assays, tumor growth assays, tumor initiation assays, etc..

Cancer cells, *in vitro* or *in vivo,* can be contacted with or supplied with the fusion peptides of the present invention. They can be directly supplied as peptides or they can be endogenously produced by supplying the cells with nucleic acid vectors which express and produce the fusion peptides in the cells. For *in vivo* administration, any delivery technique known in the art can be used, including but not limited to direct intratumoral injection, intramuscular injection, intravascular injection, subcutaneous injection, intraperitoneal injection, etc. *In vitro* delivery can be accomplished, for example, simply by supplying the fusion peptide to the culture medium.

Cancers and cancer cells which may be treated include breast, skin, colon, ovarian, prostate, cervical, colorectal, lung, brain, head and neck, pancreatic, kidney, and liver. The effect which is observed upon administration is a reduced extent or retarded rate of initiation, growth, invasion, and metastasis. Suitable assays for measuring these processes are described in the examples. Other assays as are known in the art can be used as well.

Fusion peptides can be formulated or modified as are known in the art. This may involve covalent modifications, such as capping, or PEGylation, or combination with micelles or liposomes. Such modifications and formulations may increase stability in the body, therefore permitting higher percentages of the input dosage to reach the target cancer. The fusion proteins can also be used in conjunction with other treatments, including but not limited to EGFR inhibitors. The treatments may be administered simultaneously or serially. Other suitable treatments for treating cancers include chemotherapeutic drug administration or infusion, anti-tumor antibodies, anti-receptor antibodies, radiation treatment, radiolabeled drugs, and surgery. Use of two modalities which act in different ways may provide increased benefit to the patient. Suitable EGFR inhibitors may be antibodies or kinase inhibitors, such as panitumumab, cetuximab, gefitinib, and erlotinib.

A similar inhibitory effect on the binding of MUC1 to β-catenin can be obtained by delivering antibodies to the cell or cancer patient. The antibodies can be any type, monoclonal or polyclonal, single chain or multi-chain. The antibodies may be made in a host mammal, in cell culture, or in recombinant cells. The antibodies bind to an epitope contained in SEQ ID NO: 1. Antibodies can be raised using a peptide according to SEQ ID NO: 1 as an immunogen, for example, or using fusion proteins according to the invention as immunogens, or using other fusion proteins as immunogens.

Vectors for delivery of nucleic acids encoding the fusion proteins of the present invention can be any that are known in the art. Adenoviral vectors and adeno-associated vectors are will known and widely used. Non-viral vectors can also be used, such as nanoparticles, liposomes, and micelles. Retroviral vectors can be used in some embodiments. The person of skill in the art can select a vector that is suitable for her purposes. Similarly the person of skill in the art can select a vector and host cell system for recombinant manufacture of the fusion proteins of the invention in culture.

A person who is identified as having inherited genes associated with cancer is at increased risk of developing cancer. Such persons can be treated to reduce their risk of cancer initiation. Similarly, those who have been exposed to environmental risks, such as atomic bomb fall-out, nuclear fuel waste, and other pollutants, are at increased risk of developing cancer. Genes which may be mutated and the autosomal dominant disorders they cause include, but are not limited to BRCA1: breast cancer, BRCA2: breast cancer, APC: colon cancer HNPCC: colon cancer, CDKN2: melanoma. Other autosomal dominant inherited cancer risks include basal cell nevus syndrome, neurofibromatosis type 2, Carney syndrome, osteochondromatosis, multiple, chordoma, familial, paraganglioma, familial, Cowden syndrome, Peutz-Jeghers syndrome, esophageal cancer with tylosis, prostate cancer, gastric cancer, familial, renal cancer, familial, Li-Fraumeni syndrome, retinoblastoma, multiple endocrine neoplasia type 1, tuberous sclerosis, multiple endocrine neoplasia type 2, von Hippel-Lindau disease, neurofibromatosis type 1, and Wilms' tumor. Autosomal recessive disorders disposing to cancer include ataxiatelangiectasia Rothmund-Thomson syndrome, Bloom syndrome xeroderma pigmentosa, Werner's syndrome, and Fanconi's anemia.

MUC1 mimetic peptides (MIP) linked to a protein transduction domain (PTD) can freely enter transformed cells and inhibit their invasion *in vitro.* These same peptides can inhibit primary tumor growth, tumor spread, and recurrence of tumors after resection, for example, in an orthotopically implanted breast cancer model. PMIP mimetic peptides, such as SEQ ID NO: 17 and 18, can survive in circulation with tissue specific retention and no detectable toxicity. Importantly, PMIP can significantly inhibit tumor growth, for example, in a spontaneous mouse model that mimics human breast cancer. Mechanistically, this tumor-inhibitory effect is closely associated with a decrease in EGFR and MUC1 expression. Together these data demonstrate that these peptide-based intracellular drugs show strong efficacy as non-toxic treatment for cancers.

The over-expression of human MUC1 in mouse mammary glands promotes transformation, and the loss of MUC1 in several transgenic models can significantly delay tumor onset (6, 10, 12). This may be due to the number of oncogenic partners MUC1 has been demonstrated to interact with, namely β-catenin, src, and EGFR [Reviewed in (8)]. This mimetic peptide is designed to block interactions between MUC1, β-catenin and EGFR, and we have demonstrated that PMIP treatment does block MUC1 interactions with these proteins. In addition, we observed a loss of MUC1 expression in response to PMIP treatment under certain conditions, which may be the result of downregulation of EGFR. It is tempting to speculate that in the presence of PMIP, MUC1 and EGFR are alternatively trafficked and enter the lysosomal degradation pathway, leading to their enhanced degradation. PMIP treatment of MDA-MB-231 cells overexpressing MUC1 under a CMV promoter for only 3 hours in culture induces a loss of MUC1 overexpression, indicating that the effect is not transcriptionally regulated (data not shown).

One important observation is the lack of toxicity associated with PMIP treatment (no weight loss, signs of distress or organ failure). While this is not an unexpected result with the use of an endogenous peptide, it points to a potentially low level of toxicity in patients. We have also examined the possibility that PMIP has activated an immune response in the immune-intact MMTV- pyV mT transgenic animals. Examining leukocytic infiltrates using protein levels of CD45 as a marker, we found no increase in those tumors treated with PMIP versus control ((337); data not shown). Additionally, studies by groups investigating the potential adjuvant activity of the protein transduction domain have found that the TAT protein transduction domain is not immunogenic (38).

We have recently demonstrated that MUC1 inhibits the ligand-dependent degradation of EGFR, resulting in enhanced receptor stability (9). Furthermore, we have demonstrated that this interaction promotes the oncogenic properties of EGFR (10). Neither of the mouse models used in these experiments were previously shown to be dependent upon EGFR for progression, and yet, PMIP is significantly effective in each model. This indicates that PMIP may have broad applications against tumors overexpressing MUC1, which encompasses most epithelial neoplasias (39). In addition, PMIP may serve as an important adjuvant therapy with anti-EGFR treatments [Reviewed in (40), which is expressly incorporated for this purpose]. Our data indicate that MUC1 induces the internalization, altered trafficking and enhanced signaling of EGFR (9). Therefore, if PMIP blocks these interactions, anti-EGPR therapy that relies on surface-localization of EGFR could by enhanced by PMIP co-delivery.

We have demonstrated the efficacy of PTD-linked peptide-based drugs and the value of MUC1-directed targets in cancer, especially breast cancer. Importantly, these data indicate that PMIP is a potent drug that is active at all stages of tumor progression: inhibiting initiation, inhibiting growth, inducing regression, and inhibiting metastatic spread.

### EXAMPLE 1-- Methods:

*Cell Culture.* Metastatic breast cancer cell lines MDA-MD-231 and BT20 cells were obtained from the American Tissue Culture Collection. These cell lines were cultured with 10 % Fetal Bovine Serum (Cellgro, Herndon, VA), 1 % Penicillin-Streptomycin-Glutamine (Invitrogen, Eugene, OR) and RPMI 1640 (Cellgro) media at 37° C with 5 % CO₂ in a humidified incubator.

*Invasion Assay.* Collagen matrix (0.9 mg/ml Type I Rat tail collagen (BD Biosciences, Billerica, MA), 83.0 % (v/v) M-199 medium (Life Technologies), and 0.18 % NaHCO₃ (Fisher, Hampton, NH)) was poured into a 24 well plate. Prior to collagen polymerization a 0.8 µm pore transwell insert (Coming Inc., Corning, NY) was placed on top of the matrix. The polymerized collagen was rehydrated using a chemoattractant (20 % FBS/RPMI 1640 with 100 ng/mL EGF). MDA-MB-231 cells were treated with 50µM of peptide in serum free RPMI 1640 media for 17 hours. Prior to loading the cells onto the transwell inserts they were fluorescently labeled with Calcein-AM (Invitrogen) for 30 minutes and then washed with PBS. The cells were then resuspended in a 50 µM peptide/RPMI 1640 serum free media and loaded onto the inserts in each well (266,000 cells/well). The cells were allowed to invade into the matrix for 12 hrs at 37° C with 5% CO₂ in a humidified incubator. After the 12 hrs, the collagen matrix was treated with a 0.25 % Collagenase in 40 % FBS/PBS and the inserts were removed (Calbiochem, San Diego, CA). The number of fluorescently labeled cells that had invaded into the collagen were measured using a Molecular Devices spectrophotometer (Ex:485, Em:538, and Cutoff:530).

*Immunoprecipitation and Immunoblotting.* Protein was treated as described in (9).

*Densitometry.* Immunoblotting analysis was performed as in (9).

*Antibodies and growth factors.* CT2 (anti-MUC1 cytoplasmic tail) was purchased from Neomarkers Inc. (Fremont, CA). Antibodies against phosphorylated tyrosine (PY99), and EGFR/erbB1 (1005) were all purchased from Santa Cruz Biotechnologies (Santa Cruz, CA). The β-actin antibody was from Sigma Chemical Company (St. Louis, MO). Secondary antibodies conjugated to HRP were acquired from Molecular Probes (Invitrogen) and the anti-Hamster HRP conjugated antibody was purchased from Jackson Labs (West Grover, PA). Epidermal growth factor (EGF) was stored at -20° C at a concentration of 100 ng/µl (Invitrogen).

*Immunofluorescence.* BT20 cells were treated for 2 hours with 200 µM hPMIP tagged with FITC and fixed in 4 % Paraformaldhyde/PBS. Fixed cells were washed 3 times with 0.02% NaN₃/PBS and incubated with *Slowfade* Gold antifade reagent with DAPI (Invitrogen). Cells were visualized with a fluorescence DMLB Leica compound microscope.

*Peptide Synthesis.* hPMIP (SEQ ID NO: 17), FITC-hPMIP, msPMIP (SEQ ID NO: 18), FITC-msPMIP and PTD4 polypeptides (SEQ ID NO: 16) were synthesized by GenScript (Scotch Plains, NJ) and delivered lyophilized. The peptides were resuspended at a concentration of 500 µM in PBS and stored at -80° C in single-use aliquots.

*Human Breast Tumor Xenografts.* Immunocompromised *(scid)* mice (Taconic, Rockville, MD) were tested for the presence of serum IgG and found to be <20 µg/ml IgG. Female mice (four to six weeks old) were injected with 1x10⁷ cells embedded in Matri-gel (BD Biosciences) into the mammary fat pad and allowed to grow to either 100 mm³ or 500 mm³, based on the formula *a*² x *b*/₂ where *a* is the smaller diameter and b is the larger diameter. Mice were injected intraperitoneally (i.p.) with 50 µg/g body weight of either PMIP or PTD4 control peptide for 21 days and measured with calipers every two days. Either at the end of 21 days, or after the tumor had reached 800 mm³, tumors were resected by injecting the mice with buprenorphine (2.5 mg/kg body weight, Infusion Solutions, Totowa, NJ) at least one hour prior to resection and anesthetizing the mice with isoflurane (Abbott, Abbott Park, II). Following surgery, mice were treated with buprenorphine (2.5 mg/kg body weight) 8 and 16 hrs post-surgery. Animals were then followed for 10 days to examine regrowth at the primary tumor site or at secondary mammary glands, then sacrificed.

*Transgenic Mice.* MMTV- pyV mT mice (33), obtained from Jackson Laboratories) entered into study upon the development of tumors that measured ≥ 0.5 cm in at least one diameter. Only mice greater than seven weeks of age were included in the study and mice that developed fluid cysts were excluded. Animals were injected i.p. with 50 µg/g body weight of msPMIP or PTD4 once daily for 21 days. Each of ten mammary glands were measured using calipers every two days, and measurements were used to determine tumor volume based on the formula *a*² x *b*/₂, with *a* being the smaller diameter and b being the larger diameter. After treatment for 21 days, mice were sacrificed by CO₂ inhalation and tissues resected. Several msPMIP treated mice were injected with FITC-msPMIP (50 µg/g body weight) one or fours hours prior to sacrifice and intact tissues were visualized using a fluorescence MZFLIII Leica dissection microscope.

Both the xenograft and transgenic mouse studies were performed by the Experimental Mouse Shared Services (University of Arizona, Tucson) under protocols approved by the Institutional Animal Care and Use Committee of the University of Arizona.

*Statistical Analysis.* All statistics were performed in Excel (Microsoft).

### EXAMPLE 2-

*PMIP* is *retained in cells, reduces EGFR phosphorylation and inhibits MUC1 and β-catenin interactions.* The MUC1 cytoplasmic domain is composed of 72 amino acids, within which lies a 15 amino acid domain containing sites of EGFR phosphorylation (human=YEKV and mouse=YEEV) and β-catenin binding (human=SAGNGGSSLS (SEQ ID NO: 9) and mouse=SAGNGSSSLS (SEQ ID NO: 19), Fig. 1*A*) (11, 29). We synthesized a 15 amino acid peptide to determine if it could act in a dominant negative fashion to block interactions between endogenous MUC1 and EGFR/β-catenin/src. To allow this peptide to gain entrance to the cell, we synthesized it in tandem with a protein transduction domain [PTD4, Fig. 1*A* (30), Reviewed in (31)]. Cells pulsed *in vitro* with a FITC-labeled PTD4 conjugated to MIP peptides (FITC-hPMIP) were found to contain the fluorescent peptide and it was retained over time (Fig. 1*B*).

As MUC1 interactions with β-catenin are increased during metastatic progression, we next examined the *in vitro* effects of hPMIP treatment on these interactions in the BT20 cell line (12, 32).

### EXAMPLE 3-

*PMIP inhibits invasion.* These cells were chosen as they represent models of high and low MUC1 expression, respectively (data not shown). We found that PMIP treatment significantly inhibited the ability of cells to invade through an 8.0 µM filter and into a Type I collagen matrix when compared to cells treated with control peptide (PTD4) or PBS (Fig. 1*C* and D). To determine if hPMIP could also affect proliferation and/or apoptosis we performed Annexin V and cell number quantification on the human breast cancer cell line MDA-MB-231. hPMIP treatment was found to have no detectable effects on apoptosis or cell growth when cells were grown on plastic (data not shown).

### EXAMPLE 4-

*PMIP inhibits tumor growth and inhibits recurrence in a xenograft breast cancer model.* As hPMIP strongly inhibited cellular invasion *in vitro,* we next evaluated the ability of hPMIP to suppress tumor recurrence and metastasis *in vivo.* We examined whether hPMIP could alter the metastatic potential of MDA-MB-231 breast cancer cells implanted into the mammary fat pad of severe combined immune deficiency *(scid)* mice. We found that treatment with hPMIP yielded a substantial inhibition in tumor regrowth and spread to secondary mammary glands post resection of the primary tumor (Fig. 2*B* and *2E*). In addition, we found that hPMIP treatment also slowed the growth of the primary tumor (Fig. 2*C*).

In the first experiment (Fig. 2*A* and 2*B*), cells were allowed to establish a large tumor mass (500 mm³) and mice were injected (i.p.) for 21 days with hPMIP or control peptide (PTD4) (Fig. 2A). At the end of treatment, primary breast tumors were resected, and animals were followed to examine rates of tumor regrowth and/or metastasis to secondary mammary glands. While regrowth and secondary mammary gland tumors were found in equal number for both treatment groups, the tumor volume for the control treated animals averaged 760 mm³ while the PMIP treated averaged only 73 mm³ (Fig. 2*B*). Note that mice were not treated with drug during the 10 days in which regrowth was followed. We also noted a decrease in the tumor size in the hPMIP-treated animals compared to control, and next designed an experiment that would allow us to determine if hPMIP was affecting tumor growth rate in this model.

To determine potential effects of hPMIP on primary tumor growth, we repeated the MDA-MB-231 xenograft experiment (21 days of drug treatment), but began treatment at a smaller tumor size (100 mm³) (Fig. 2*C*-2*E*). We allowed the tumors to continue to grow after 21 days of drug treatment and performed surgical resection of the primary tumors when they reached a volume of 800 mm³, which allowed us to also evaluate tumor spread in this experiment (Fig. 2D). Treatment with hPMIP resulted in a significant decrease in tumor size compared to control treated animals (Fig. 2C, p=0.028). This corresponded to a significant increase in the length of time required for hPMIP treated mice to reach resection size of 800 mm³ (Fig. 2*D*, p=0.03). Although treatment ended approximately 20 days prior to resection, we observed that hPMIP treatment substantially decreased the amount of tumor regrowth and spread 10 days after resection (Fig. *2E*). Together, these data demonstrated that hPMIP treatment can inhibit tumor growth, spread and recurrence in a highly metastatic breast cancer model.

### EXAMPLE 5

*PMIP inhibits, tumor growth and induces regression in spontaneous breast cancer.* While the xenograft model demonstrated the effect of hPMIP treatment on growth and progression of established cell lines, we wanted to determine how msPMIP would affect tumor initiation and progression in a mouse model which better recapitulates human breast cancer. The MMTV-pyV mT transgenic mouse model of breast cancer strongly resembles human breast cancer by activating multiple signaling pathways, including AKT, src and shc (33, 34). Studies have demonstrated that the resulting breast cancer pathologically and molecularly mimics the full progression of hyperplasia, ductal carcinoma *in situ* and adenocarcinomas observed in human disease (35, 36). To determine if peptide could be delivered to the mammary glands and tumors of these animals, we injected FITC-labeled msPMIP and analyzed peptide retention (Fig. 3A). At one hour post-injection, FITC was detected throughout the animals body cavity, including all organs (data not shown). After four hours, FITC-msPMIP was found to be retained selectively in the mammary gland tumor and in the colon and skin (Fig. 3*A* and data not shown).

To determine the effects of msPMIP on spontaneous breast cancer progression, MMTV-pyV mT mice bearing mammary gland tumors of >0.5cm in diameter were treated for 21 days with either msPMIP or PTD4 control peptide. Treatment had a dramatic effect on tumor growth, as msPMIP significantly slowed the total tumor growth from ∼590 % to ∼194% over the 21 days of treatment (p=0.039; Fig. 3*B*). Additionally, msPMIP treatment significantly decreased the tumor growth rate to only 25 mm³/day compared to 69 mm³/day in control (PTD4) treated tumors (p=0.007; Fig. 3*C*). Note that treatment of MMTV-pyV mT mice with hPMIP (as opposed to msPMIP) had no effect on tumor growth, emphasizing the amino acid specificity of PMIP.

We next analyzed the overall size of tumors that arose throughout the study. This analysis demonstrates that while 13% of the tumors in the control (PTD4) group grew larger than 500 mm³ by the end of the study, only 1% of the tumors in the msPMIP treated group reached that size (Fig. 3D). As this transgenic model had continual expression of the polyoma middle T transgene driving tumorigenesis throughout the study, we next examined the effects of drug treatment on the formation of new tumors. Although both msPMIP and control (PTD4) groups had a similar number of tumors sized 100-300 mm³ at the beginning of treatment, this number doubled by the end of treatment in the control group, but remained the same in the msPMIP group (Fig. 3D). These data indicated that msPMIP treatment inhibits tumor initiation in this model. To analyze tumor initiation further, we evaluated the percent of tumors that were initiated during drug treatment (Initiation equals percent of tumor transitions from 0 mm³ to 100 mm³). This analysis demonstrates that in the msPMIP group there was a significant decrease (p=0.0045; Fig. 3*E*) of tumor initiation during the study.

Although highly significant decreases in tumor formation and growth were observed from treatment of tumor-bearing MMTV-pyV mT mice with msPMIP, not all tumors in the study responded to treatment (Fig. 4). Analysis of each mammary gland demonstrates that while most tumor growth rates slowed substantially in response to msPMIP, a number of tumors continued to grow, indicative of the stochastic pathway activation in this model. Importantly, a subset of established tumors treated with msPMIP (four tumors) regressed completely under treatment, although none of the control treated tumors did so (Fig. 4, *).

### EXAMPLE 6-

*msPMIP treatment results in a reduction of EGFR and Mucl levels.* Previously, we observed that MUC1 expression inhibits ligand-dependent degradation of EGFR, while others demonstrated that the SAGNGGSSLS sequence facilitates MUC1/β-catenin interactions (9, 11). To determine if msPMIP was affecting EGF-dependent degradation of EGFR, we generated protein lysates from tumors of MMTV- pyV mT animals injected with EGF and peptide 30 minutes prior to animal sacrifice (after a standard 21 day drug treatment). We observed a striking reduction in the expression of EGFR and corresponding phosphotyrosine in the msPMIP treated mouse compared to the control treated animal (Fig. 5*A*). Note that in this mouse, there were no frank tumors remaining after the 21 day msPMIP treatment, while we obtained six tumors of greater than 400 mm³ from control treated animals.

To determine if msPMIP blocked interaction between Muc1 and β-catenin, we began by establishing levels of Muc1 protein expression in the tumor lysates. Interestingly, we found that msPMIP treatment induced a loss of Muc1 protein expression in both the MMTV- pyV mT model (Fig. 5*A* and 5*B*) and in the MDA-MB-231 xenograft model (Fig. 5*C* and 5*D*). While the mechanism of this loss is unknown, it would certainly result in a loss of Muc1-dependent oncogenic signaling.

### References

1. Hilkens, J., Vos, H.L., Wesseling, J., Boer, M., Storm, J., van der Valk, S., Calafat, J., and Patriarca, C. 1995. Is episialin/MUC1 involved in breast cancer progression? Cancer Lett 90:27-33.
2. Zotter, S., Hageman, P.C., Lossnitzer, A., Mooi, W.J., and Hilgers, J. 1988. Tissue and tumor distribution of human polymorphic epithelial mucin. Cancer Reviews 11-12:55-101.
3. Brossart, P., Schneider, A., Dill, P., Schammann, T., Grunebach, F., Wirths, S., Kanz, L., Buhring, H.J., and Brugger, W. 2001. The epithelial tumor antigen MUC1 is expressed in hematological malignancies and is recognized by MUC1-specific cytotoxic T-lymphocytes. Cancer Res 61:6846-6850.
4. Takahashi, T., Makiguchi, Y., Hinoda, Y., Kakiuchi, H., Nakagawa, N., Imai, K., and Yachi, A. 1994. Expression of MUC1 on myeloma cells and induction of HLA-unrestricted CTL against MUC1 from a multiple myeloma patient. J Immunol 153:2102-2109.
5. Teruya-Feldstein, J., Donnelly, G.B., Goy, A., Hegde, A., Nanjangud, G., Qin, J., Thaler, H., Gilles, F., Dyomin, V.G., Lloyd, K.O., et al. 2003. MUC-1 mucin protein expression in B-cell lymphomas. Appl Immunohistochem Mol Morphol 11:28-32.
6. Schroeder, J.A., Masri, A.A., Adriance, M.C., Tessier, J.C., Kotlarczyk, K.L., Thompson, M.C., and Gendler, S.J. 2004. MUC1 overexpression results in mammary gland tumorigenesis and prolonged alveolar differentiation. Oncogene 23:5739-5747.
7. Ren, J., Agata, N., Chen, D., Li, Y., Yu, W.H., Huang, L., Raina, D., Chen, W., Kharbanda, S., and Kufe, D. 2004. Human MUC1 carcinoma-associated protein confers resistance to genotoxic anticancer agents. Cancer Cell 5:163-175.
8. Hollingsworth, M.A., and Swanson, B.J. 2004. Mucins in cancer: protection and control of the cell surface. Nat Rev Cancer 4:45-60.
9. Pochampalli, M.R., el Bejjani, R.M., and Schroeder, J.A. 2007. MUC1 is a novel regulator of ErbB1 receptor trafficking. Oncogene 26:1693-1701.
10. Pochampalli, M.R., Bitler, B.G., and Schroeder, J.A. 2007. Transforming Growth Factor alpha-Dependent Cancer Progression is Modulated by Muc1 Cancer Research 67*.*
11. Li, Y., Ren, J., Yu, W., Li, Q., Kuwahara, H., Yin, L., Carraway, K.L., 3rd, and Kufe, D. 2001. The epidermal growth factor receptor regulates interaction of the human DF3/MUC1 carcinoma antigen with c-Src and beta-catenin. J Biol Chem 276:35239-35242.
12. Schroeder, J.A., Adriance, M.C., Thompson, M.C., Camenisch, T.D., and Gendler, S.J. 2003. MUC1 alters ß-catenin-dependent tumor formation and promotes cellular invasion. Oncogene 22:1324-1332.
13. Yamamoto, M., Bharti, A., Li, Y., and Kufe, D. 1997. Interaction of the DF3/MUC1 breast carcinoma-associated antigen and beta-catenin in cell adhesion. J Biol Chem 272:12492-12494.
14. Li, Y., Bharti, A., Chen, D., Gong, J., and Kufe, D. 1998. Interaction of glycogen synthase kinase 3beta with the DF3/MUC1 carcinoma-associated antigen and beta-catenin. Mol Cell Biol 18:7216-7224.
15. Li, Y., Kuwahara, H., Ren, J., Wen, G., and Kufe, D. 2001. The c-Src tyrosine kinase regulates signaling of the human DF3/MUC1 carcinoma-associated antigen with GSK3 beta and beta-catenin. J Biol Chem 276:6061-6064.
16. Schroeder, J.A., and Lee, D.C. 1997. Transgenic mice reveal roles for TGFalpha and EGF receptor in mammary gland development and neoplasia. J Mammary Gland Biol Neoplasia 2:119-129.
17. Alroy, I., and Yarden, Y. 1997. The ErbB signaling network in embryogenesis and oncogenesis: signal diversification through combinatorial ligand-receptor interactions. FEBS Lett 410:83-86.
18. Olayioye, M.A., Graus-Porta, D., Beerli, R.R., Rohrer, J., Gay, B., and Hynes, N.E. 1998. ErbB-1 and ErbB-2 acquire distinct signaling properties dependent upon their dimerization partner. Mol Cell Biol 18:5042-5051.
19. Olayioye, M.A., Beuvink, I., Horsch, K., Daly, J.M., and Hynes, N.E. 1999. ErbB receptor-induced activation of stat transcription factors is mediated by Src tyrosine kinases. J Biol Chem 274:17209-17218.
20. Carpenter, G. 2000. The EGF receptor: a nexus for trafficking and signaling. Bioessays 22:697-707.
21. Schroeder, J.A., Thompson, M.C., Gardner, M.M., and Gendler, S.J. 2001. Transgenic MUC1 interacts with epidermal growth factor receptor and correlates with mitogenactivated protein kinase activation in the mouse mammary gland. J Biol Chem 276:13057-13064.
22. Schroeder, J.A., Troyer, K.L., and Lee, D.C. 2000. Cooperative induction of mammary tumorigenesis by TGFalpha and Wnts. Oncogene 19:3193-3199.
23. Polakis, P. 2000. Wnt signaling and cancer. Genes Dev 14:1837-1851.
24. He, T.C., Sparks, A.B., Rago, C., Hermeking, H., Zawel, L., da Costa, L.T., Morin, P.J., Vogelstein, B., and Kinzler, K.W. 1998. Identification of c-MYC as a target of the APC pathway. Science 281:1509-1512.
25. Shtutman, M., Zhurinsky, J., Simcha, I., Albanese, C., D'Amico, M., Pestell, R., and Ben-Ze'ev, A. 1999. The cyclin D1 gene is a target of the beta-catenin/LEF-1 pathway. Proc Natl Acad Sci USA 96:5522-5527.
26. Tetsu, O., and McCormick, F. 1999. Beta-catenin regulates expression of cyclin D1 in colon carcinoma cells. Nature 398:422-426.
27. Tsukamoto, A.S., Grosschedl, R., Guzman, R.C., Parslow, T., and Varmus, H.E. 1988. Expression of the int-1 gene in transgenic mice is associated with mammary gland hyperplasia and adenocarcinomas in male and female mice. Cell 55:619-625.
28. Michaelson, J.S., and Leder, P. 2001. beta-catenin is a downstream effector of Wnt-mediated tumorigenesis in the mammary gland. Oncogene 20:5093-5099.
29. Spicer, A.P., Duhig, T., Chilton, B.S., and Gendler, S.J. 1995. Analysis of mammalian MUC1 genes reveals potential functionally important domains. Mamm Genome 6:885-888.
30. Ho, A., Schwarze, S.R., Mermelstein, S.J., Waksman, G., and Dowdy, S.F. 2001. Synthetic protein transduction domains: enhanced transduction potential in vitro and in vivo. Cancer Res 61:474-477.
31. Wadia, J.S., and Dowdy, S.F. 2005. Transmembrane delivery of protein and peptide drugs by TAT-mediated transduction in the treatment of cancer. Adv Drug Deliv Rev 57:579-596.
32. Schroeder, J.A., Adriance, M.C., McConnell, E.J., Thompson, M.C., Pockaj, B.A., and Gendler, S.J. 2002. ErbB/beta -catenin complexes are associated with human infiltrating ductal breast and MMTV-Wnt-1 and MMTV-c-neu transgenic carcinomas. J Biol Chem 277:22692-22698.
33. Guy, C.T., Cardiff, R.D., and Muller, W.J. 1992. Induction of mammary tumors by expression of polyomavirus middle T oncogene: a transgenic mouse model for metastatic disease. Mol Cell Biol 12:954-961*.*
34. Webster, M.A., Hutchinson, J.N., Rauh, M.J., Muthuswamy, S.K., Anton, M., Tortorice, C.G., Cardiff, R.D., Graham, F.L., Hassell, J.A., and Muller, W.J. 1998. Requirement for both Shc and phosphatidylinositol 3' kinase signaling pathways in polyomavirus middle T-mediated mammary tumorigenesis. Mol Cell Biol 18:2344-2359.
35. Maglione, J.E., Moghanaki, D., Young, L.J., Manner, C.K., Ellies, L.G., Joseph, S.O., Nicholson, B., Cardiff, R.D., and MacLeod, C.L. 2001. Transgenic Polyoma middle-T mice model premalignant mammary disease. Cancer Res 61:8298-8305.
36. Lin, E.Y., Jones, J.G., Li, P., Zhu, L., Whitney, K.D., Muller, W.J., and Pollard, J.W. 2003. Progression to malignancy in the polyoma middle T oncoprotein mouse breast cancer model provides a reliable model for human diseases. Am J Pathol 163:2113-2126.
37. Trivedi, P., Cuomo, L., Christensson, B., Hu, L.F., Morrone, S., Frati, L., Faggioni, A., Winberg, G., and Klein, G. 2000. Augmentation of leukocyte infiltration in murine tumors expressing B-cell derived but not nasopharyngeal carcinoma derived EBV membrane protein LMP1. J Med Virol 60:417-424.
38. Kittiworakam, J., Lecoq, A., Moine, G., Thai, R., Lajeunesse, E., Drevet, P., Vidaud, C., Menez, A., and Leonetti, M. 2005. HIV-1 Tat Raises an Adjuvant-free Humoral Immune Response Controlled by its Core Region and its Ability to Form Cysteine-mediated Oligomers. The Journal of Biological Chemistry 281:3105-3115.
39. Packer, L.M., Williams, S.J., Callaghan, S., Gotley, D.C., and McGuckin, M.A. 2004. Expression of the cell surface mucin gene family in adenocarcinomas. Int J Oncol 25:1119-1126.
40. Dassonville, O., Bozec, A., Fischel, J.L., and Milano, G. 2007. EGFR targeting therapies: monoclonal antibodies versus tyrosine kinase inhibitors. Similarities and differences. Crit Rev Oncol Hematol 62:53-61.
41. Schroeder, J. et al., U.S. Patent Application US-2006-0293234-A1.

## Claims

1. A fusion peptide having a structure:
A-B-C or C-B-A
for use in treating a human who has an identified elevated risk of cancer,
wherein A is a protein transduction domain selected from the group consisting of SEQ ID NO: 3 to 6, which enhances translocation of attached macromolecules across cellular membranes;
wherein B is a spacer of 0-5 amino acid residues;
wherein C is a polypeptide of 6-15 amino acid residues, wherein C comprises all or a portion of PYEKVSAGNGGSSLS (SEQ ID NO: 1), and wherein the portion of C comprises GGSSLS (SEQ ID NO: 2), or wherein at least one of said 6-15 amino acid residues is substituted with an alanine amino acid residue provided the invasion and metastasis inhibiting activity is maintained.

2. The fusion protein for use according to claim 1 wherein:
(1) the identified elevated risk is due to a genetic predisposition;
(2) the identified elevated risk is due to environmental exposure; or
(3) the identified elevated risk is due to occupational exposure.

3. The fusion protein for use according to claim 1 wherein:
(1) the human has an elevated risk due to a genetic predisposition to breast cancer;
(2) the human has an elevated risk due to a genetic predisposition to colon cancer; or
(3) the human has an elevated risk due to a genetic predisposition to skin cancer.

4. The fusion protein for use according to claim 1 wherein at least one of said 6-15 amino acid residues is substituted with an alanine amino acid residue.

5. The fusion protein for use according to claim 1 wherein C comprises PYEKVSAGNGGSSLS (SEQ ID NO: 1).

6. A fusion peptide having a structure:
A-B-C or C-B-A
and an EGFR inhibitor, for use in treating a human who has had a tumor resected,
wherein A is a protein transduction domain selected from the group consisting of SEQ ID NO: 3 to 6, which enhances translocation of attached macromolecules across cellular membranes;
wherein B is a spacer of 0-5 amino acid residues;
wherein C is a polypeptide of 6-15 amino acid residues, wherein C comprises all or a portion of PYEKVSAGNGGSSLS (SEQ ID NO: 1), and wherein the portion of C comprises GGSSLS (SEQ ID NO: 2), or wherein at least one of said 6-15 amino acid residues is substituted with an alanine amino acid residue provided the invasion and metastasis inhibiting activity is maintained.

7. The fusion protein and EGFR inhibitor for use according to claim 6 wherein at least one of said 6-15 amino acid residues is substituted with an alanine amino acid residue.

8. The fusion protein and EGFR inhibitor for use according to claim 6 wherein the EGFR inhibitor is panitumumab, cetuximab, gefitinib or erlotinib.

9. The fusion protein and EGFR inhibitor for use according to claim 6, wherein C comprises PYEKVSAGNGGSSLS (SEQ ID NO: 1).

10. A fusion peptide having a structure:
A-B-C or C-B-A
for use in treating a patient with skin cancer,
wherein A is a protein transduction domain selected from the group consisting of SEQ ID NO: 3 to 6, which enhances translocation of attached macromolecules across cellular membranes;
wherein B is a spacer of 0-5 amino acid residues;
wherein C is a polypeptide of 6-15 amino acid residues, wherein C comprises all or a portion of PYEKVSAGNGGSSLS (SEQ ID NO: 1), and wherein the portion of C comprises GGSSLS (SEQ ID NO: 2), or wherein at least one of said 6-15 amino acid residues is substituted with an alanine amino acid residue provided the invasion and metastasis inhibiting activity is maintained.

11. The fusion protein for use according to claim 10 wherein:
(1) at least one of said 6-15 amino acid residues is substituted with an alanine amino acid residue; or
(2) C comprises PYEKVSAGNGGSSLS (SEQ ID NO: 1).

## Patentansprüche

1. Ein Fusionspeptid mit der Struktur
A-B-C oder C-B-A
zur Verwendung bei der Behandlung eines Menschen, der ein identifiziertes erhöhtes Krebsrisiko aufweist, worin A eine Proteintransduktionsdomäne ist, ausgewählt aus der Gruppe, bestehend aus der Sequenz SEQ ID Nr. 3 bis 6, welches die Translokation von gebundenen Makromolekülen durch die Zellmembranen steigert,
worin C ein Spacer von 0 bis 5 Aminosäureresten ist,
worin C ein Polypeptid von 6 bis 15 Aminosäureresten ist, worin C gänzlich oder einen Teil von PYEKVSAGNGGSSLS (SEQ ID Nr. 1) umfasst und worin der Anteil von C GGSSLS (SEQ ID Nr. 2) umfasst oder worin mindestens eine der 6 bis 15 Aminosäureresten mit einem Alanin als Aminosäurerest substituiert ist, vorausgesetzt die Invasion und Metastase inhibierende Wirkung erhalten bleibt.

2. Das Fusionsprotein zur Verwendung nach Anspruch 1, worin
(1) das identifizierte erhöhte Risiko durch eine genetische Prädisposition,
(2) das identifizierte erhöhte Risiko durch Umwelteinflüsse oder
(3) das identifizierte erhöhte Risiko aufgrund beruflicher Exposition hervorgerufen ist.

3. Das Fusionsprotein zur Verwendung nach Anspruch 1, worin
(1) der Mensch ein erhöhtes Risiko aufgrund einer genetischen Prädisposition für Brustkrebs,
(2) der Mensch ein erhöhtes Risiko aufgrund einer genetischen Prädisposition für Kolonkrebs, oder
(3) der Mensch ein erhöhtes Risiko aufgrund einer genetischen Prädisposition für Hautkrebs aufweist.

4. Das Fusionsprotein zur Verwendung nach Anspruch 1, worin mindestens eine der 6 bis 15 Aminosäureresten mit einem Alanin als Aminosäurerest substituiert ist.

5. Das Fusionsprotein zur Verwendung nach Anspruch 1, worin C PYEKVSAGNGG SLS (SEQ ID Nr. 1) umfasst.

6. Ein Fusionspeptid mit der Struktur
A-B-C oder C-B-A
und einen EGFR-Inhibitor zur Verwendung bei der Behandlung eines Menschen, dem ein Tumor herausgeschnitten wurde, worin A eine Proteintransduktionsdomäne, ausgewählt aus der Gruppe, bestehend aus der SEQ ID Nr. 3 bis 6, die die Translokation von gebundenen Makromolekülen durch die Zellmembranen steigert,
worin B ein Spacer von 0 bis 5 Aminosäureresten ist,
worin C ein Polypeptid von 6 bis 15 Aminosäureresten ist, worin C gänzlich oder teilweise PYEKVSAGNGGSSLS (SEQ ID Nr. 1) und worin der Anteil von C GGSSLS (SEQ ID Nr. 2) umfasst oder worin mindestens eine der 6 bis 15 Aminosäurereste mit einem Alanin als Aminosäurerest substituiert ist, vorausgesetzt die Invasion und Metastase inhibierende Wirkung erhalten bleibt.

7. Das Fusionsprotein und EGFR-Inhibitor zur Verwendung nach Anspruch 6, worin mindestens eine der 6 bis 15 Aminosäurereste mit einem Alanin als Aminosäurerest substituiert ist.

8. Das Fusionsprotein und EGFR-Inhibitor zur Verwendung nach Anspruch 6, worin der EGFR-Inhibitor Panitumumab, Cetuximab, Gefitinib oder Erlotinib ist.

9. Das Fusionsprotein und EGFR-Inhibitor zur Verwendung nach Anspruch 6, worin C PYEKVSAGNGGSSLS (SEQ ID Nr. 1) umfasst.

10. Ein Fusionspeptid mit der Struktur
A-B-C oder C-B-A
zur Behandlung eines Patienten mit Hautkrebs,
worin A eine Proteintransduktionsdomäne ist, ausgewählt aus der Gruppe, bestehend aus SEQ ID Nr. 3 bis 6, die die Translokation von gebundenen Makromolekülen durch die Zellmembranen verstärkt, worin B ein Spacer von 0 bis 5 Aminosäureresten ist, worin C eine Polypeptid von 5 bis 15 Aminosäureresten ist, worin C alles oder ein Teil von PYEKVSAGNGGSSLS (SEQ ID Nr. 1) und worin der Anteil von C GGSSLS (SEQ ID Nr. 2) ist oder worin mindestens einer der 6 bis 15 Aminosäurereste mit einem Alanin als Aminosäurerest substituiert ist, vorausgesetzt die Invasion und Metastase inhibierende Wirkung erhalten bleibt.

11. Das Fusionsprotein zur Verwendung nach Anspruch 10, worin
(1) mindestens eine der 6 bis 15 Aminosäurereste mit einem Alanin als Aminosäurerest substituiert ist oder
(2) C PYEKVSAGNGGSSLS (SEQ ID Nr. 1) umfasst.

## Revendications

1. Peptide de fusion de structure :
A-B-C ou C-B-A
destiné à être utilisé pour le traitement d'un humain chez qui on a identifié un risque élevé de cancer,
dans lequel A est un domaine de transduction d'une protéine choisi dans le groupe formé par SEQ ID NO : 3 à 6 qui augmente la translocation de macromolécules liées à travers les membranes cellulaires,
B est un élément d'espacement renfermant 0-5 résidus d'acide aminé, C est un polypeptide renfermant 6-15 résidus d'acide aminé, C renfermant la totalité ou une partie de PYEKVSAGNGGSSLS (SEQ ID NO : 1) et la partie de C comprenant GGSSLS (SEQ ID NO : 2), ou au moins l'un des 6-15 résidus d'acide aminé étant substitué par un résidu de l'acide aminé alanine,
à la condition que l'activité d'inhibition de l'invasion et des métastases soit maintenue.

2. Protéine de fusion destinée à être utilisée conformément à la revendication 1, dans laquelle :
(1) le risque élevé identifié est causé par une prédisposition génétique,
(2) le risque élevé identifié est causé par une exposition environnementale, ou
(3) risque élevé identifié est causé par une exposition liée à la profession.

3. Protéine de fusion destinée à être utilisée conformément à la revendication 1, dans laquelle :
(1) l'humain a un risque élevé causé par une prédisposition génétique au cancer du sein,
(2) l'humain a un risque élevé causé par une prédisposition génétique au cancer du colon, ou
(3) l'humain a un risque élevé causé par une prédisposition génétique au cancer de la peau.

4. Protéine de fusion destinée à être utilisée conformément à la revendication 1, dans laquelle au moins l'un des 6-15 résidus d'acide aminé est substitué par un résidu de l'acide aminé alanine.

5. Protéine de fusion destinée à être utilisée conformément à la revendication 1, dans laquelle C renferme PYEKVSAGNGGSSLS (SEQ ID NO : 1).

6. Protéine de fusion de structure :
A-B-C ou C-B-A,
et inhibiteur de l'EGFR destinés à être utilisés pour le traitement d'un humain ayant eu une résection de tumeur,
protéine dans laquelle A est un domaine de transduction d'une protéine choisi dans le groupe formé par SEQ ID NO : 3 à 6 qui augmente la translocation de macromolécules liées à travers les membranes cellulaires,
B est un élément d'espacement renfermant 0-5 résidus d'acide aminé, C est un polypeptide renfermant 6-15 résidus d'acide aminé, C renfermant la totalité ou une partie de PYEKVSAGNGGSSLS (SEQ ID NO : 1) et la partie de C comprenant GGSSLS (SEQ ID NO : 2), ou, au moins l'un des 6-15 résidus d'acide aminé étant substitué par un résidu de l'acide aminé alanine,
à la condition que l'activité d'inhibition de l'invasion et des métastases soit maintenue.

7. Protéine de fusion et inhibiteur de l'EGFR destinés à être utilisés conformément à la revendication 6, dans lesquels au moins l'un des 6-15 résidus d'acide aminé est substitué par un résidu de l'acide aminé alanine.

8. Protéine de fusion et inhibiteur de l'EGFR destinés à être utilisés conformément à la revendication 6, dans lesquels l'inhibiteur de l'EGFR est l'un des inhibiteurs suivants : panitumumab, cetuximab, gefitinib ou erlotinib.

9. Protéine de fusion et inhibiteur de l'EGFR destinés à être utilisés conformément à la revendication 6, dans lesquels C renferme PYEKVSAGNGGSSLS (SEQ ID NO : 1).

10. Peptide de fusion de structure :
A-B-C ou C-B-A,
destiné à être utilisé pour le traitement d'un patient présentant un cancer de la peau,
dans lequel A est un domaine de transduction d'une protéine choisi dans le groupe formé par SEQ ID NO : 3 à 6 qui augmente la translocation de macromolécules liées à travers les membranes cellulaires,
B est un élément d'espacement renfermant 0-5 résidus d'acide aminé, C est un polypeptide renfermant 6-15 résidus d'acide aminé, C renfermant la totalité ou une partie de PYEKVSAGNGGSSLS (SEQ ID NO : 1) et la partie de C comprenant GGSSLS (SEQ ID NO : 2), ou, au moins l'un des 6-15 résidus d'acide aminé étant substitué par un résidu de l'acide aminé alanine,
à la condition que l'activité d'inhibition de l'invasion et des métastases soit maintenue.

11. Protéine de fusion destinée à être utilisée conformément à la revendication 10, dans laquelle :
(1) au moins l'un des 6-15 résidus d'acide aminé est substitué par un résidu de l'acide aminé alanine, ou
(2) C renferme PYEKVSAGNGGSSLS (SEQ ID NO : 1).
